Europäisches Patentamt

(19) European Patent Office

Office européen des brevets

(11) Veröffentlichungsnummer: **0 000 891**
**B1**

(12) **EUROPÄISCHE PATENTSCHRIFT**

(21) Anmeldenummer: **78100603.6**

(22) Anmeldetag: **07.08.78**

(51) Int. Cl.³: **A 61 K 31/43**

(54) Synergistische Kombinationen von Penicillinen und sie enthaltende antibiotische Mittel.

(30) Priorität: **20.08.77 DE 2737673**

(43) Veröffentlichungstag der Anmeldung:
**07.03.79 Patentblatt 79/05**

(45) Bekanntmachung des Hinweises auf die Patenterteilung:
**09.01.80 Patentblatt 80/01**

(84) Benannte Vertragsstaaten: **CH DE FR GB**

(56) Entgegenhaltungen:
**DE – A – 2 540 523**
**GB – A – 1 175 590**

(73) Patentinhaber: **BAYER Aktiengesellschaft,
Zentralbereich Patente, Marken und Lizenzen
Bayerwerk,
D–5090 Leverkusen 1 (DE)**

(72) Erfinder: **Metzger, Karl Georg, Dr.,
Pahlkestrasse 15
D–5600 Wuppertal 1 (DE)**

Synergistische Kombinationen von Penicillinen und sie enthaltende antibiotische Mittel.

Die Erfindung betrifft neue antibiotische synergistische Wirkstoffkombinationen aus bekannten Penicillinen. Die Wirkstoffkombinationen bestehen aus zwei Wirkstoffgruppen. Die Wirkstoffe der ersten Gruppe (Gruppe A) sind die bekannten Penicilline D-α-[(Imidazolidin-2-on-1-yl)-carbonylamino]-benzylpenicillin (US Patent 3 933 795 ) und D-α-[(3-Methylsulfonyl-imidazolidin-2-on-1-yl)-carbonylamino]-benzylpenicillin (Deutsche Offenlegungsschrift 2 152 967). Die Wirkstoffe der zweiten Gruppe (Gruppe B) sind die bekannten Penicilline 3-(2,6-Dichlorphenyl)-5-methyl-4-isoxazolylpenicillin, 3-(2-Chlor-6-fluorphenyl)-5-methyl-4-isoxazolylpenicillin, 3-(2-Chlorphenyl)-5-methyl-4-isoxazolylpenicillin, 3-Phenyl-5-methyl-4-isoxazolylpenicillin und 2,6-Dimethoxyphenylpenicillin.

Die erfindungsgemäßen synergistischen Wirkstoffkombinationen sind dadurch gekennzeichnet, daß sie mindestens ein Penicillin der Wirkstoffgruppe A und mindestens ein Penicillin der Wirkstoffgruppe B enthalten.

Es ist bekannt, daß Breitspektrumpenicilline wie α-Carboxy-, α-Amino-benzylpenicillin oder α-Carboxy-3-thienylmethylpenicillin gegenüber bestimmten Bakterienarten unwirksam oder wenig wirksam sind, weil diese Bakterien den β-Lactamring spaltende Enzyme entwickeln. Es ist ferner bekannt, daß die Zugabe von Penicillinen der Wirkstoffgruppe B, die gegenüber der Einwirkung von β-Lactamasen stabil sind, zu einem Synergismus führen kann. Dieser gibt sich dadurch zu erkennen, daß die Resistenz der β-Lactamase bildenden Bakterien gegenüber beispielsweise α-Carboxy- oder α-Aminobenzylpenicillin durch die Zugabe von Penicillinen der Wirkstoffgruppe B aufgehoben oder stark vermindert wird (siehe beispielsweise Deutsche Offenlegungsschrift 2 062 938). Solche synergistischen Effekte wurden bisher jedoch beispielsweise nicht bei Bakterien der Gattung der β-Lactamase-bildenden Klebsiellen beobachtet, weil Ampicillin als gegenüber Klebsiellen nicht oder sehr wenig wirksam gilt.

Überraschenderweise wurde nun erstmals gefunden, daß die erfindungsgemäßen neuen Wirkstoffkombinationen gegen diese Bakteriengattungen einen starken synergistischen Effekt aufzuweisen haben. Dieses ist umsomehr überraschend, als die Penicilline der erfindungsgemäßen Wirkstoffgruppe A auf Bakterien sehr viel weniger lysierend wirken als beispielsweise α-Aminobenzylpenicillin.

In der erfindungsgemäßen Kombination der Wirkstoffe der Gruppe A mit denen der Gruppe B können die Wirkstoffe in der Säure- oder Salzform sowie in den möglichen Kristall- und Hydratformen vorliegen.

Das molare Verhältnis der Wirkstoffe der Gruppe A zu denen der Gruppe B kann im Bereich von 9 : 1 bis 1 : 9 liegen. Es ist vorzugsweise im Bereich von 3 : 1 bis 1 : 3 und ganz besonders bevorzugt 1 : 1.

Von den Wirkstoffen der Gruppe A ist das Penicillin D-α-[(3-Methylsulfonyl-imidazolidin-2-on-1-yl)-carbonylamino]-benzylpenicillin bevorzugt. Von den Wirkstoffen der Gruppe B ist das Penicillin 3-Phenyl-5-methyl-4-isoxazolylpenicillin bevorzugt.

Sofern Wirkstoffe der Gruppe A oder B als Salze vorliegen, sind dieses pharmazeutisch verwendbare Salze der sauren Carboxylgruppe dieser Wirkstoffe mit anorganischen oder organischen Basen.

Als Basen können hierzu alle in der pharmazeutischen Chemie, insbesondere in der Chemie der Antibiotika, üblicherweise verwendeten Basen eingesetzt werden. Als anorganische Basen seien beispielhaft genannt: Alkali- und Erdalkalihydroxide, Alkali- und Erdalkalicarbonate und Alkalihydrogencarbonate, wie Natrium- und Kaliumhydroxid, Calcium- und Magnesiumhydroxid, Natrium- und Kaliumcarbonat, Calciumcarbonat, Natrium- und Kaliumhydrogencarbonat; Aluminiumhydroxid und Ammoniumhydroxid. Als organische Amine können primäre, sekundäre und tertiäre aliphatische Amine sowie heterocyclische Amine eingesetzt werden. Beispielhaft seien genannt: Di- und Triniedrigalkylamine, z.B. Diäthylamin, Triäthylamin, Tri-β-hydroxyäthylamin, Procain, Dibenzylamin, N,N'-Di-benzyläthylendiamin, N-Benzyl-β-phenyl-äthylamin, N-Methyl- und N-Äthylmorpholin, l-Ephenamin, Dehydroabietylamin, N,N'-Bis-dehydroabietyläthylendiamin, N-Niedrigalkylpiperidin.

Auch sogenannte basische Aminosäuren wie Lysin oder Arginin können vorteilhaft als Basen Verwendung finden. Besonders bevorzugte Salze sind die Natriumsalze.

Die erfindungsgemäßen synergistischen Wirkstoffgemische weisen bei geringer Toxizität eine starke und breite antimikrobielle Wirksamkeit auf. Diese Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen synergistischen Wirkstoffgemische sind gegen ein sehr breites Spektrum von Mikroorganismen wirksam. Mit ihrer Hilfe können gramnegative und grampositive Bakterien und bakterienähnliche Mikroorganismen erstmals unproblematisch bekämpft sowie die durch diese Erreger hervorgerufenen Erkrankungen verhindert, gebessert und/oder geheilt werden.

Besonders wirksam sind die erfindungsgemäßen synergistischen Wirkstoffgemische gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischung der folgenden Erreger verursacht werden:

Enterobacteriaceae, wie Escherichiae-Bakterien der Coli-Gruppe: Escherichia-Bakterien, z.B. Escherichia coli, Klebsiella-Bakterien, z.B. K.pneumoniae, K.pneumoniae, Proteae-Bakterien der Proteus-Gruppe: Proteus, z.B. Proteus vulgaris, Pr.morganii, Pr.rettgeri.

Die obige Aufzählung von Erregern ist lediglich beispielhaft und keineswegs beschränkend aufzufassen.

Als Krankheiten, die durch die erfindungsgemäßen synergistischen Wirkstoffgemische verhindert, gebessert und/oder geheilt werden können, seinen beispielsweise genannt: Erkrankungen der Atmungswege und des Rachenraumes; Otitis; Pharyngitis; Pneumonie; Peritonitis; Pyelonephritis; Cystitis; Endocarditis; Systeminfektionen; Bronchitis; Arthritis; lokale Infektionen.

Zur vorliegenden Erfindung gehören pharmazeutische Zubereitungen, die neben inerten pharmazeutisch geeigneten Träger- und/oder Zusatzstoffen eine erfindungsgemäße Wirkstoffkombination enthalten oder die aus einer erfindungsgemäßen Wirkstoffkombination bestehen.

Die therapeutisch wirksamen synergistischen Wirkstoffgemische sollen in den oben aufgeführten pharmazeutischen Zubereitungen vorzugsweise in einer Gesamtkonzentration von etwa 0,1 bis 99,5, vorzugsweise von etwa 0,5 bis 95 Gewichtsprozent der Gesamtmischung vorhanden sein.

Die oben aufgeführten pharmazeutischen Zubereitungen können außer den erfindungsgemäßen Wirkstoffgemischen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die synergistischen Wirkstoffgemische oder ihre pharmazeutischen Zubereitungen können lokal, oral, parenteral, intraperitoneal und/oder rectal, vorzugsweise oral oder parenteral wie intravenös oder intramuskulär appliziert werden.

Im allgemeinen hat es sich sowohl in der Humanals auch in der Veterinärmedizin als vorteilhaft erwiesen, die erfindungsgemäßen Wirkstoffe in Gesamtmengen von etwa 5 bis etwa 1000, vorzugsweise 20 bis 200 mg/kg Körpergewicht je 24 Stunden, gegebenenfalls in Form mehrerer Einzelgaben, zur Erzielung der gewünschten Ergebnisse zu verabreichen. Eine Einzelgabe enthält das erfindungsgemäße Wirkstoffgemisch, vorzugsweise in Mengen von etwa 1 bis etwa 250, insbesondere 10 bis 100 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoffgemisch auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen. Im Falle der intravenösen Anwendung enthält eine Ampulle beispielsweise 2 g D-$\alpha$-[(3-Methylsulfonyl-imidazolidin-2-on-1-yl)-carbonylamino]-benzylpenicillin und 1 g 3-Phenyl-5-methyl-4-isoxazolylpenicillin. Im Falle der Anwendung als Futterzusatzmittel können die neuen synergistischen Wirkstoffgemische in den üblichen Konzentrationen und Zubereitungen zusammen mit dem Futter bzw. mit Futterzubereitungen oder mit dem Trinkwasser gegeben werden. Dadurch kann eine Infektion durch gramnegative oder grampositive Bakterien verhindert, gebessert und/oder geheilt werden und ebenso eine Förderung des Wachstums und eine Verbesserung der Verwertung des Futters erreicht werden.

Die neuen synergistischen Wirkstoffgemische zeichnen sich durch starke antibakterielle Wirkungen, die in vivo und in vitro geprüft wurden, und durch orale Resorbierbarkeit aus.

Die Wirksamkeit der erfindungsgemäßen Wirkstoffkombinationen sei durch die folgende Tabelle erläutert:

Minimale Hemmkonzentration in mcg/ml:

| Keim: | 1 | 2 | 3 | 4 | 5 | Substanzen: 1+3 | 1+4 | 2+3 | 2+4 | 5+3 | 5+4 |
|---|---|---|---|---|---|---|---|---|---|---|---|
| Proteus morg. 1102 | >512 | >512 | >512 | >512 | >256 | 32+32 | 32+32 | – | – | >256 | >256 |
| Proteus mirabilis υ 468 | >512 | >512 | 512 | 512 | >256 | 256+256 | 128+128 | >256+256 | >256+256 | >256+256 | >256+256 |
| Proteus rettgeri 985 | 128 | >512 | >512 | 512 | >256 | 64+64 | 64+64 | 256+256 | 256+256 | >256+256 | 256+256 |
| E.coli υ 250 | 128 | 256 | 512 | 512 | >256 | 32+32 | 32+32 | 128+128 | 128+128 | >256+256 | 256+256 |
| E.coli 1465 | 256 | >512 | >512 | 512 | >256 | 128+128 | 128+128 | >256+256 | >256+256 | >256+256 | >256+256 |
| Proteus morganii υ 1306 | 2 | 32 | 512 | >512 | 512 | – | 1+1 | 16+16 | 1+1 | 64+64 | 32+32 |
| Proteus morganii 1272 | 16 | 128 | >512 | >512 | >256 | 16+16 | 8+8 | 64+64 | 0,5+0,5 | 64+64 | 32+32 |
| Klebsiella υ 1673 | 64 | 256 | 256 | 512 | 512 | 32+32 | 32+32 | 128 | 256 | ./. | 256 |

Erläuterung:
Substanz 1 = D-$\alpha$-[(3-Methylsulfonyl-imidazolidin-2-on-1-yl)-carbonylamino]-benzyl-penicillin.
Substanz 2 = D-$\alpha$-[(Imidazolidin-2-on-1-yl)-carbonylamino]-benzylpenicillin.
Substanz 3 = 3-Phenyl-5-methyl-4-isoxazolylpenicillin.
Substanz 4 = 3-(2,6-Dichlorphenyl)-5-methyl-4-isoxazolylpenicillin.
Substanz 5 = $\alpha$-Amino-benzylpenicillin.

Die minimalen Hemmwerte (MHK) der Tabelle wurden in Muller-Hinton-Nährbrühe im Reihenverdünnungstest bei einer Einsaat von $2 \cdot 10^5$ Bakterien pro ml nach 24 Stunden Bebrütung bestimmt. Bei der Prüfung der Wirkstoffgemische war in jedem Teströhrchen das Gewichtsverhältnis von Substanz 1, 2 oder 5 zu den Substanzen 3 oder 4 wie 1 : 1. Rechnet man die Gewichtsmengen in Einheiten (IU) um, dann ergeben sich für z.B. 1 mg Substanz 5 (abgerundet) = 1700 E und für Substanz 1 = 1100 E/ml.

Die Differenz zugunsten von Substanz 1 erhöht sich also um den Faktor 1,5 im Vergleich zu Substanz 5.

**Patentansprüche**

1. Wirkstoffkombination, bestehend aus

(A) mindestens einem Penicillin aus der Gruppe D-α-[(Imidazolidin-2-on-1-yl)-carbonylamino]-benzyl-penicillin, D-α-[(3-Methylsulfonyl-imidazolidin-2-on-1-yl)-carbonylamino]-benzylpenicillin und/oder deren pharmazeutisch verwendbaren Salzen, und

(B) mindestens einem Penicillin aus der Gruppe 3-(2,6-Dichlorphenyl)-5-methyl-4-isoxazolyl-penicillin, 3-(2-Chlor-6-fluorphenyl)-5-methyl-4-isoxazolyl-penicillin, 3-(2-Chlorphenyl)-5-methyl-4-isoxazolyl-penicillin, 3-Phenyl-5-methyl-4-isoxazolyl-penicillin, 2,6-Dimethoxyphenylpenicillin und/oder deren pharmazeutisch verwendbaren Salzen.

2. Wirkstoffkombination gemäß Anspruch 1, bestehend aus D-α-[(3-Methylsulfonyl-imidazolidin-2-on-1-yl)-carbonyl-amino]-benzylpenicillin und 3-Phenyl-5-methyl-4-isoxazolyl-penicillin.

3. Wirkstoffkombination gemäß Anspruch 1, bestehend aus D-α-[(3-Methylsulfonyl-imidazolidin-2-on-1-yl)-carbonylamino]-benzylpenicillin und 3-(2,6-Dichlorphenyl)-5-methyl-4-isoxazolylpenicillin.

4. Wirkstoffkombination gemäß Anspruch 1, bestehend aus D-α-[(Imidazolidin-2-on-1-yl)-carbonylamino]-benzylpenicillin und 3-Phenyl-5-methyl-4-isoxazolylpenicillin.

5. Wirkstoffkombination gemäß Anspruch 1, bestehend aus D-α-[(Imidazolidin-2-on-1-yl)-carbonylamino]-benzylpenicillin und 3-(2,6-Dichlorphenyl)-5-methyl-4-isoxazolylpenicillin.

6. Wirkstoffkombination gemäß den Ansprüchen 1 bis 5, dadurch gekennzeichnet, daß die Wirkstoffe in Form der Natriumsalze vorliegen.

7. Wirkstoffkombination gemäß den Ansprüchen 1 bis 6, dadurch gekennzeichnet, daß das Gewichtsverhältnis von (A) zu (B) im Bereich von 9 zu 1 bis 1 zu 9, vorzugsweise im Bereich von 3 zu 1 bis 1 zu 3 liegt.

8. Wirkstoffkombination gemäß Anspruch 7, bei dem das Gewichtsverhältnis von (A) zu (B) 1 zu 1 ist.

9. Antibiotisches Mittel, gekennzeichnet durch einen Gehalt an einer Wirkstoffkombination gemäß Anspruch 1.

**Claims**

1. An active compound combination consisting of

(A) at least one penicillin from the following group: D-α-[imidazolidin-2-on-1-yl]-carbonylamino]-benzyl-penicillin, D-α-[(3-methylsulphonyl-imidazolidin-2-on-1-yl)-carbonylamino]-benzylpenicillin and/or the salts thereof which can be used pharmaceutically and (B) at least one penicillin from the following group: 3-(2,6-dichlorophenyl)-5-methyl-4-isoxazolyl-penicillin, 3-(2-chloro-6-fluorophenyl)-5-methyl-4-isoxazolyl-penicillin, 3-(2-chlorophenyl)-5-methyl-4-isoxazolyl-penicillin, 3-phenyl-5-methyl-4-isoxazolyl-penicillin, 2,6-dimethoxyphenylpenicillin and/or the salts thereof which can be used pharmaceutically.

2. An active compound combination according to claim 1, consisting of D-α-[(3-methylsulphonyl-imidazolidin-2-on-1-yl)-carbonylamino]-benzylpenicillin and 3-phenyl-5-methyl-4-isoxazolylpenicillin.

3. An active compound combination according to claim 1, consisting of D-α-[(3-methylsulphonyl-imidazolidin-2-on-1-yl)-carbonylamino]-benzylpenicillin and 3-(2,6-dichlorophenyl)-5-methyl-4-isoxazolyl-penicillin.

4. An active compound combination according to claim 1, consisting of D-α-[(imidazolidin-2-on-1-yl)-carbonylamino]-benzylpenicillin and 3-phenyl-5-methyl-4-isoxazolylpenicillin.

5. An active compound combination according to claim 1, consisting of D-α-[(imidazolidin-2-on-1-yl)-carbonylamino]-benzylpenicillin and 3-(2,6-dichlorophenyl)-5-methyl-4-isoxazolylpenicillin.

6. An active compound combination according to claims 1 to 5, characterised in that the active compounds are in the form of the sodium salts.

7. An active compound combination according to claims 1 to 6, characterised in that the weight ratio of (A) to (B) is in the range from 9 : 1 to 1 : 9, preferably in the range from 3 : 1 to 1 : 3.

8. An active compound combination according to claim 7, in which the weight ratio of (A) to (B) is 1 : 1.

9. An antibiotic agent, characterised in that it contains an active compound combination according to claim 1.

**Revendications**

1. Mélange de substances actives à effet combiné, caractérisé en ce qu'il renferme:

(A) au moins une pénicilline choisie dans le groupe comprenant la D-α-[(imidazolidine-2-one-1-yl)-carbonylamino]-benzylpénicilline, la D-α-[(3-méthylsulfonylimidazolidine-2-one-1-yl)-carbonylamino]-benzylpénicilline et/ou leurs sels acceptables du point de vue pharmaceutique et

(B) au moins une pénicilline choisie dans le groupe comprenant la 3-(2,6-dichlorphényl)-5-méthyl-4-isoxazolylpénicilline, la 3-(2-chloro-6-fluorophényl)-5-méthyl-4-isoxazolylpénicilline, la 3-(2-chlorophényl)-5-méthyl-4-isoxazolylpénicilline, la 3-phényl-5-méthyl-4-isoxazolylpénicilline, la 2,6-diméthoxyphényl-pénicilline et/ou leurs sels acceptables du point de vue pharmaceutique.

2. Mélange de substances actives suivant la revendication 1, caractérisé en ce qu'il est formé de D-α-[(3-méthylsulfonylimidazolidine-2-one-1-yl)-carbonylamino]-benzylpénicilline et de 3-phényl-5-méthyl-4-isoxazolylpénicilline.

3. Mélange de substances actives suivant la revendication 1, caractérisé en ce qu'il est formé de D-α-[(3-méthylsulfonylimidazolidine-2-one-1-yl)-carbonylamino]-benzylpénicilline et de 3-(2,6-dichlorophényl)-5-méthyl-4-isoxazolylpénicilline.

4. Mélange de substances actives suivant la revendication 1, caractérisé en ce qu'il est formé de D-α-[(imidazolidine-2-one-1-yl)-carbonylamino]-benzylpénicilline et de 3-phényl-5-méthyl-4-isoxazolylpénicilline.

5. Mélange de substances actives suivant la revendication 1, caractérisé en ce qu'il est formé de D-α-[(imidazolidine-2-one-1-yl)-carbonylamino]-benzylpénicilline et de 3-(2,6-dichlorophényl)-5-méthyl-4-isoxazolylpénicilline.

6. Mélange de substances actives suivant l'une quelconque des revendications 1 à 5, caractérisé en ce que les substances actives sont présentes sous la forme des sels de sodium.

7. Mélange de substances actives suivant l'une quelconque des revendications 1 à 6, caractérisé en ce que le rapport en poids du composant (A) au composant (B) est compris dans la plage de 9 : 1 à 1 : 9 et de préférence dans la plage de 3 : 1 à 1 : 3.

8. Mélange de substances actives suivant la revendication 7, caractérisé en ce que le rapport en poids du composant (A) au composant (B) est égal à 1 : 1.

9. Composition antibiotique, caractérisée en ce qu'elle contient un mélange de substances actives à effet combiné suivant la revendication 1.